# FASCICULE DE BREVET EUROPEEN

(11) **EP 1 023 273 B1**
(45) Date de publication et mention de la délivrance du brevet: **05.06.2002**
(21) Numéro de dépôt: 98949063.6
(22) Date de dépôt: 14.10.1998
(51) Int. Cl.: C07D 253/06, A61K 31/53, C07D 405/12, C07D 403/12

(54) **DERIVES CYCLOHEXANIQUES DIFONCTIONNALISES EN 1,4 EN TANT QUE LIGANDS DES RECEPTEURS 5HT1A**
1,4-DISUBSTITUIERTE CYCLOHEXAN-DERIVATE ALS LIGANDEN DER 5HT1A REZEPTOREN
CYCLOHEXANE DERIVATIVES DIFUNCTIONALISED IN 1,4 AS LIGANDS OF 5HT1A RECEPTORS

(30) Priorité: 16.10.1997 FR 9712954
(43) Date de publication de la demande: 02.08.2000
(73) Titulaire: PIERRE FABRE MEDICAMENT, 92100 Boulogne (FR)
(72) Inventeur: PATOISEAU, Jean-François, F-81100 Castres (FR); DUPONT-PASSELAIGUE, Elisabeth, F-81100 Castres (FR); KOEK, Wouter, F-81290 Viviers-les-Montagnes (FR)
(74) Mandataire: Ahner, Francis
(86) Numéro de dépôt international: FR9802207
(87) Numéro de publication internationale: WO9920613

(56) Documents cités:
- WO-A-95/01965
- WO-A-96/16949

## Description

La présente invention a pour objet de nouveaux dérivés cyclohexaniques fonctionnalisés en 1,4, leur préparation et leur application en thérapeutique humaine.

Les récepteurs 5-HT_{1A} ont été revendiqués pour leur rôle dans différentes pathologies telles que l'hypertension, le dysfonctionnement sexuel, l'anorexie, la mémoire. La principale cible suggérant l'implication des récepteurs 5-HT_{1A} est cependant constituée par les troubles du système nerveux central tels que l'anxiété et la dépression. Les hypothèses, appuyées par des tests sur modèles animaux et des études cliniques suggèrent que des traitements plus efficaces de ces pathologies peuvent être envisagés avec des composés agonistes 5-HT_{1A} de forte affinité et possédant une grande sélectivité et une forte efficacité.

Des dérivés de la 3,5-dioxo-(*2H, 4H*)-1,2,4-triazine et de la 3,5-dioxo-6-amino (*2H,4H*)-1,2,4-triazine ont été revendiqués précédemment par la demanderesse (FR.2.707.294 du 06/07/93 et FR.2.727.682 du 02/12/94).

Les composés de la présente invention se caractérisent par leur puissante affinité vis-à-vis du récepteur 5-HT_{1A} associée à une grande sélectivité, notamment vis-à-vis des récepteurs D₂ et α₁, et une forte activité intrinsèque.

Les composés de l'invention correspondent à la formule générale I 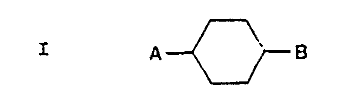 dans laquelle
- A représente un groupement de formule 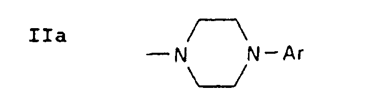 dans lequel Ar représente lui-même une structure de type aromatique choisie parmi phényle ou pyrimidinyle, éventuellement substituée par un ou plusieurs groupements alcoyle en C₁₋₃, alcoxy en C₁-C₃, trifluorométhyle ou halogène. 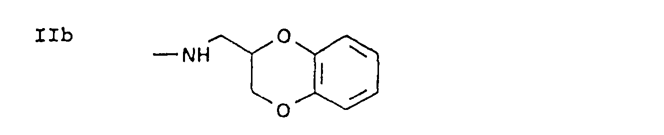
- B représente un groupement hétérocyclique de formules
   . 3,5-dioxo-(*2H*,*4H*)-1,2,4 triazine substitué en position 2(IIIa) 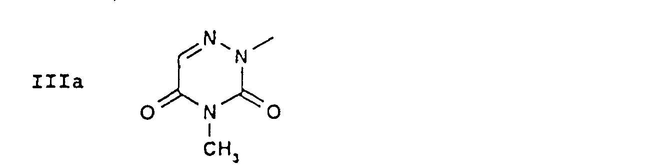
   . 3-oxo-(*2H*)-1,2,4 triazine substitué en position 5 (IIIb) 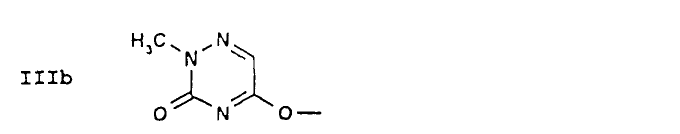 ou bien
   . 3,5-dioxo-6-amine-(*2H*,*4H*)-1,2,4-triazine (IIIc) 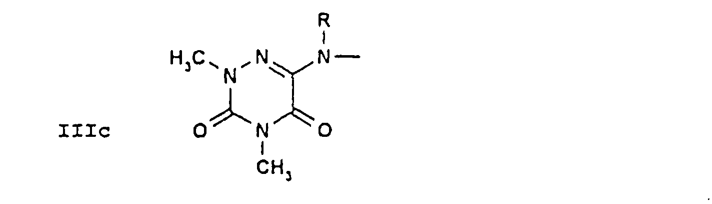
   dans lequel R représente un groupement alcoyle en C₁-C₃.

L'invention couvre les sels des composés de formule générale I avec les acides pharmaceutiquement acceptables. Elle couvre en outre les différents isomères « cis » et « trans » ainsi que les différents énantiomères des composés possédant des carbones asymétriques.

### SYNTHESE

Les composés de la présente invention peuvent être synthétisés en utilisant les voies de synthèse décrites ci-dessous ou en utilisant des méthodes de synthèse connues de l'homme de métier.

### Méthode 1

La synthèse des composés de formule générale I où B représente un groupement IIIa ou IIIb est caractérisée en ce que l'on condense un dérivé de formule générale IV 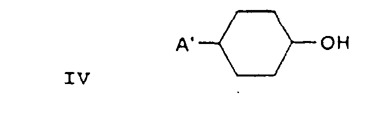 dans lequel A' représente les groupements IIa ou IIb décrits précédemment ou IIc 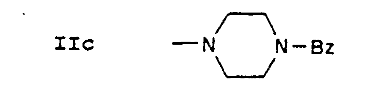 avec un intermédiaire de type Va ou Vb

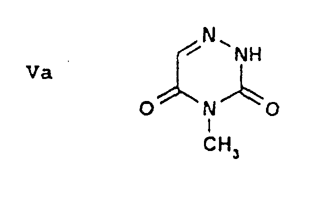

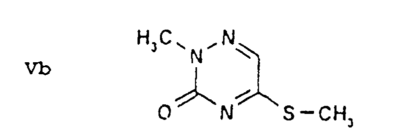

L'intermédiaire IV dans lequel A' = IIc est utilisé avantageusement dans le cas du couplage avec les dérivés de type Va pour accéder aux composés I dans lesquels A représente IIa avec Ar représentant un groupement pyrimidinyle éventuellement substitué. Le composé de condensation entre les intermédiaires IV dans lequel A' = IIc et Va est débenzylé en traitant par exemple avec le chloroformiate d'α-chloro éthyle dans le méthanol puis en condensant sur la chloro-2-pyrimidine éventuellement substituée en présence d'une base telle que la triéthylamine dans le toluène.

La condensation entre les dérivés IV et Va peut être effectuée selon les conditions de la réaction de Mitsunobu.

La condensation entre les dérivés IV et Vb peut être effectuée en présence d'une base telle que l'hydrure de sodium ou le tertiobutylate de potassium dans le dioxane ou le THF.

### Méthode 2

La synthèse des composés de formule générale I où B représente un groupement IIIc selon la méthode 2 est caractérisée en ce que l'on traite un dérivé de formule générale VI 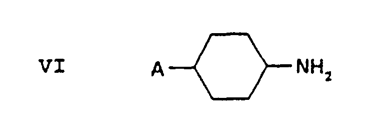 dans lequel A a la même signification que précédemment avec un intermédiaire de formule générale Vc

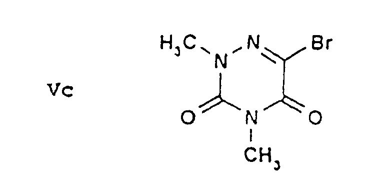

La condensation est effectuée en présence d'une base telle que la triéthylamine dans le butanol.

La séparation éventuelle des énantiomères de composés obtenus selon la méthode 1 ou la méthode 2 et possédant un carbone asymétrique est généralement réalisée sur les produits terminaux par chromatographie liquide sur colonne chirale.

### Synthèse des alcools IV

a) Lorsque A' représente un groupe IIa ou IIc 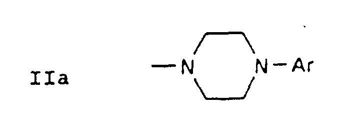 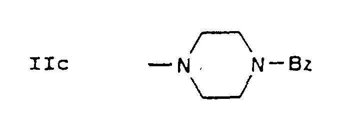 les alcools correspondants IVa ou IVc 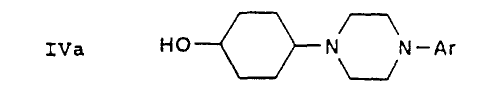 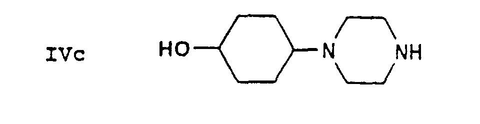 peuvent être obtenus en condensant la pipérazine VIIa ou VIIb 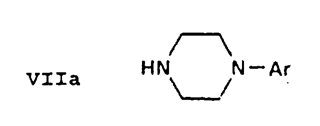 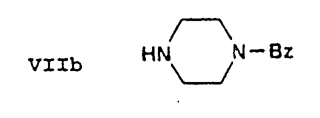 sur la cyclohexanedione monoéthylène cétal en présence d'un réducteur tel que NaBH(OAc), dans le dichlorométhane ou NaBH₃CN dans l'éthanol pour conduire à l'intermédiaire VIIIa ou VIIIb 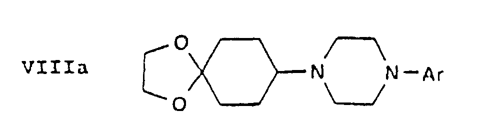 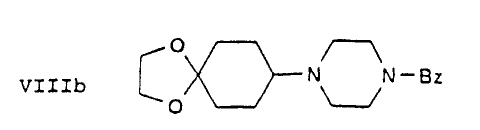 qui est hydrolysé dans un milieu aqueux tel que l'acide chlorhydrique pour donner la cétone IXa ou IXb. 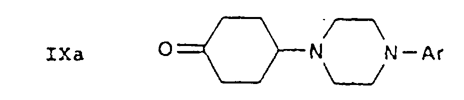 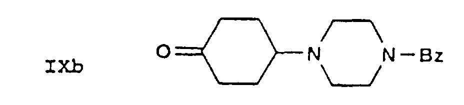
   La cétone IXa est réduite en alcool IVa par des agents réducteurs tels que NaBH₄ dans l'éthanol pour accéder majoritairement aux alcools IVa « trans » ou le L-S-sélectride dans le THF pour conduire majoritairement aux alcools IVa « cis ».
   La cétone IXb, réduite de façon analogue à celle décrite ci-dessus puis débenzylée en traitant par exemple avec le chloroformiate d'α-chloroéthyle dans le méthanol fournit l'alcool IVc. Cet alcool, condensé sur la 2-chloropyrimidine éventuellement substituée dans un solvant tel que le toluène en présence d'une base telle que la triéthylamine fournit l'alcool IVa avec Ar représentant un groupement de type pyrimidinyle.
b) lorsque A' représente un groupement IIb 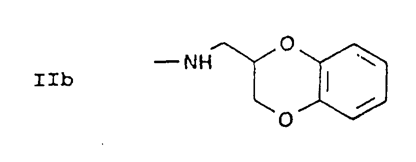 l'alcool IVb peut être obtenu en condensant l'amine X 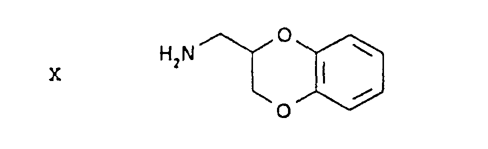 sur la cyclohexanedione monoéthylène cétal en présence d'un réducteur tel que NaBH(OAc), dans le dichlorométhane ou NaBH₃CN dans l'éthanol pour conduire à l'intermédiaire VIIIc 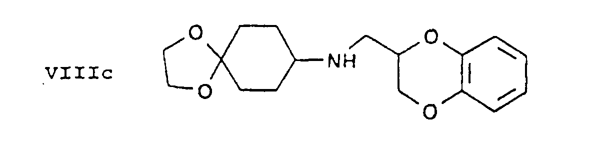 qui est hydrolysé en milieu acide chlorhydrique aqueux pour donner la cétone IXc.

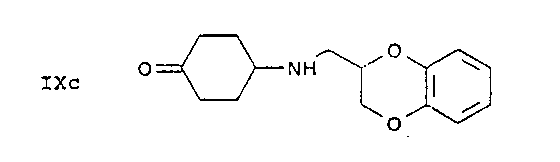

La cétone IXc est réduite en alcool IVb par des agents réducteurs tels que NaBH₄ dans l'éthanol pour accéder majoritairement aux composés IVb « trans » ou le L-S-sélectride dans le THF pour conduire majoritairement aux alcools IVb « cis ».

### Synthèse des amines VI

Les amines VI peuvent être obtenues à partir des cétones correspondantes IX,

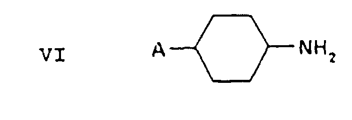

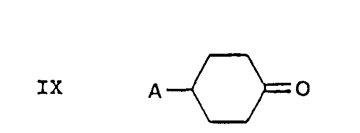

A ayant la même signification que précédemment, en les traitant à l'hydroxylamine en milieu hydroalcoolique pour conduire aux imines XI 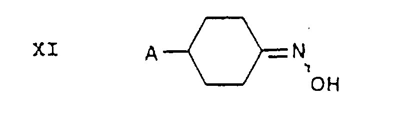 qui sont réduites par un hydrure tel que LiAlH₄ dans le THF.

### Synthèse des intermédiaires V

Les composés Va et Vc sont synthétisés selon les méthodes décrites précédemment par la demanderesse, respectivement dans les brevets FR 2.727.682 du 02/12/94 et FR.2.707.294 du 06/07/93.

Les composés Vb peuvent être obtenus selon le procédé (schéma 1) caractérisé par les étapes suivantes :
*1* - Condensation de l'acide glyoxylique sur le thiosemicarbazide, suivie d'un traitement basique tel que la soude,
*2* - Méthylation par l'iodure de méthyle en milieu basique aqueux suivi d'un traitement acide tel que l'acide chlorhydrique,
*3* - Sulfuration de la position 5 en présence du réactif de Lawesson dans un solvant tel que la pyridine,
*4* - Méthylation par l'iodure de méthyle en milieu basique aqueux tel que la soude,
*5* - Méthylation de la position 2 par l'iodure de méthyle en présence de NaH dans le DMF.

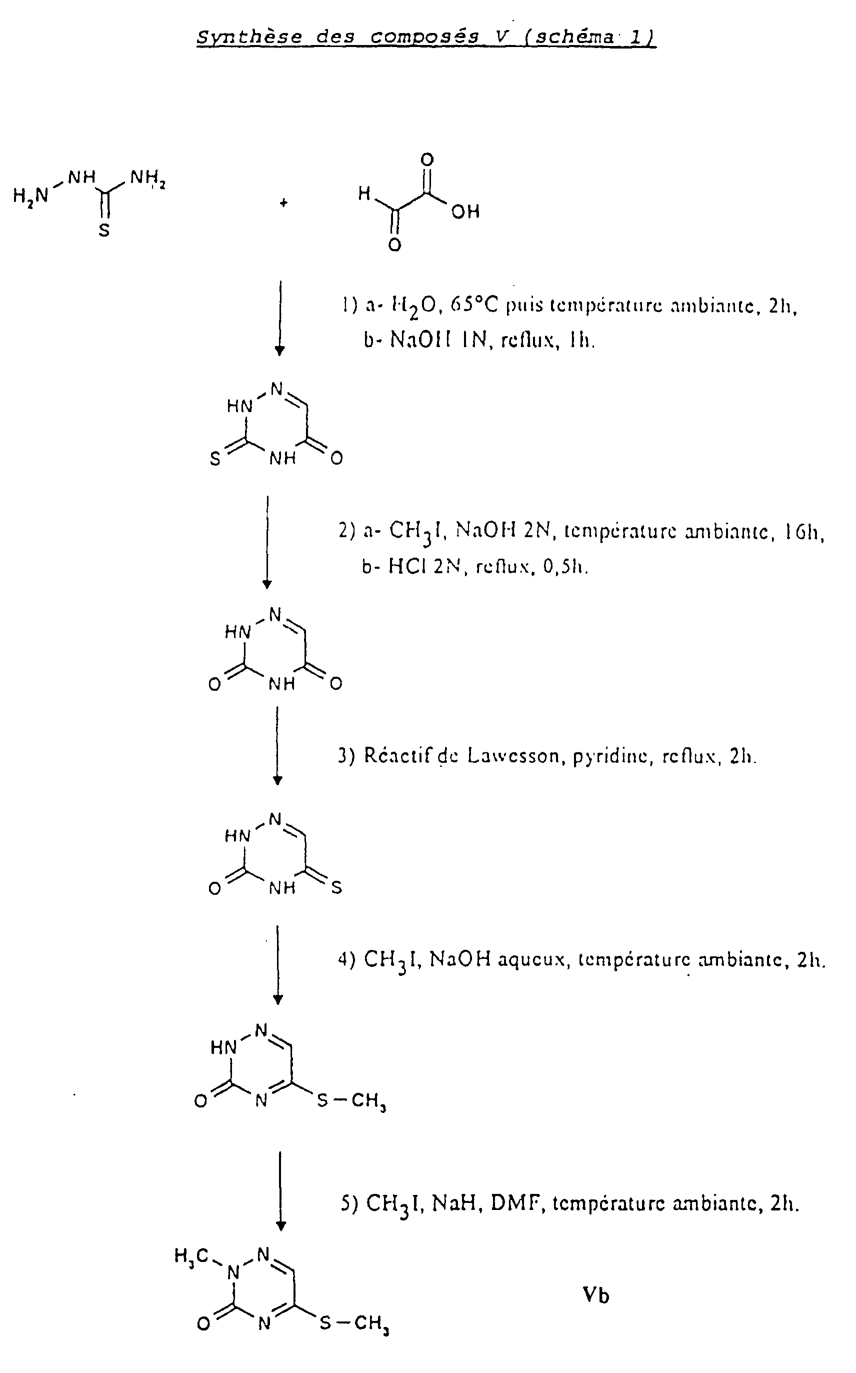

Les exemples suivants illustrent l'invention sans en limiter la portée.

Les analyses élémentaires et les spectres IR et RMN confirment les structures des composés obtenus selon l'invention.

### Exemple 1 : Cis 2-[4-[4-(3-chlore-phényl)-pipérazin-1-yl]-cyclohexyl]-4-méthyl-2H- [1,2,4]triazine-3,5-dione fumarate (1).

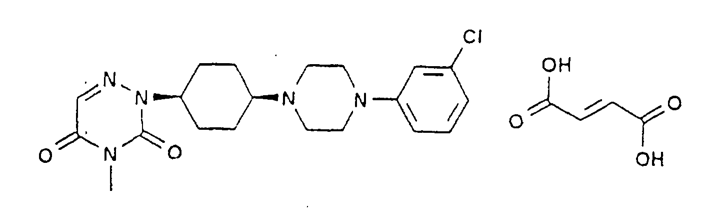

### a) 2-Acétyl-2H-[1,2,4]triazine-3,5-dione (1a).

La 2*H*-[1,2,4]triazin-3,5-dione (100 g ; 884 mmol) est placée dans 600 ml d'anhydride acétique à reflux pendant 1,5 h. Le milieu réactionnel est ensuite concentré à sec et le solide obtenu est empâté dans le toluène. Après filtration et rinçage au toluène, on isole 130,5 g de cristaux beiges.
F = 148°C
CCM gel de silice 60 F 254 Merck
CH₂Cl₂ - MeOH : 90-10 Rf = 0,68.

### b) 4-Méthyl-2H-[1,2,4]triazine-3,5-dione (1b).

NaH (60 % dans la paraffine ; 7 g ; 175 mmol) est placé en suspension dans le DMF sous atmosphère inerte. Le composé **1a** (25 g ; 161 mmol) dilué dans 200 ml de DMF est lentement coulé goutte à goutte. Le milieu réactionnel est agité 1 h à température ambiante puis CH₃I (15 ml ; 241 mmol) est additionné. Ce mélange est agité 3 h à température ambiante.

Après concentration à sec du milieu réactionnel, le résidu obtenu est repris par 190 ml d'éthanol auquel est ajouté 1,5 g d'acide paratoluène sulfonique. Ce mélange est placé 5 h à reflux puis le solvant est évaporé sous vide.

L'huile obtenue est reprise avec H₂O puis extraite au dichlorométhane. Les phases organiques sont séchées sur MgSO₄ puis concentrées. Le précipité qui se forme est filtré, lavé à l'éther. On isole 10,5 g de cristaux jaunes.
F = 180°C
CCM gel de silice 60 F 254 Merck
CH₂Cl₂ - AcOEt : 70-30 Rf = 0,46.

### c) 1-(3-Chlorophényl)-4-(1,4-dioxa-spiro[4,5]dec-8-yl)-pipérazine (1c).

La cyclohexanedione monoéthylènecétal (18,3g ; 117,2 mmol) est placée dans 45 ml de Ti(OiPr), en. présence de 3-chlorophénylpipérazine (23 g ; 117 mmol). Ce mélange est agité 2 h à température ambiante puis 110 ml d'éthanol absolu sont additionnés suivis de 6 g de NaBH₃CN. Le milieu réactionnel est agité 20 h à température ambiante.

Après neutralisation à l'aide de 24 ml d'eau, les sels de titane sont éliminés par filtration. Le filtrat est concentré à sec et l'huile obtenue est purifiée par chromatographie flash sur silice (éluent CH₂Cl₂ - AcOEt : 70 - 30) . On recueille 29,6 g d'huile (**1c**).
CCM gel de silice 60 F 254 Merck
CH₂Cl₂ - AcOEt : 70 - 30 Rf = 0,18.

### d) 4-[4-(3-Chloro-phényl)-pipérazin-1-yl]-cyclohexanone (1d).

Le composé **1c** (29,6 g ; 88 mmol) est placé dans 195 ml de dioxane en présence de 125 ml d'une solution 6N d'acide chlorhydrique puis le mélange est agité 22 h à température ambiante.

Le milieu réactionnel est neutralisé avec NaOH puis extrait avec l'acétate d'éthyle. Les phases organiques sont séchées sur MgSO₄ puis concentrées à sec. L'huile obtenue (23,5 g) est utilisée sans autre purification pour l'étape suivante.
CCM gel de silice 60 F 254 Merck
CH₂Cl₂ - MeOH : 95-5 Rf = 0,36.

### e) Trans 4-[4-(3-chlorophényl)-pipérazin-1-yl]-cyclohexanol (1e).

LiAlH₄ (3,4 g; 89,8 mmol) est placé en suspension dans 90 ml de THF sous atmosphère inerte. Le composé **1d** dilué dans 41 ml de THF est additionné goutte à goutte, puis le mélange est porté à reflux pendant 2 h.

Après neutralisation à l'aide de 13 ml d'eau, le milieu réactionnel est séché sur MgSO₄. Les minéraux sont filtrés et le filtrat est concentré à sec. Le résidu obtenu est purifié par chromatographie flash sur silice (CH₂Cl₂ - MeOH : 90 - 10). On recueille 5,7 g d'huile.
CCM gel de silice 60 F 254 Merck
CH₂Cl₂ - MeOH : 90 - 10 Rf = 0,28.

### f) Cis 2-[4-[4-(3-chloro-phényl)-pipérazin-1-yl]-cyclohexyl]-4-méthyl-2H-[1,2,4]tria-zine-3,5-dione fumarate (1).

L'alcool **1e** (0,96g ; 3,25 mmol) est placé en présence de triphénylphosphine (0,86 g ; 3,27 mmol) et de composé **1b** ( 0,46 g ; 3,62 mmol) dans 12 ml de THF sous atmosphère inerte et refroidi à 0°C sur lit de glace. Le DEAD (0,51 ml ; 3,23 mmol) est additionné goutte à goutte puis la température du milieu réactionnel est remontée à l'ambiante. Ce mélange est agité 24 h à cette température, puis concentré à sec et le résidu obtenu est purifié par chromatographie flash sur silice (éluent CH₂Cl₂ - AcOEt: 70 - 30).

Après salification avec l'acide fumarique dans l'éthanol, on isole 0,16 g de solide blanc.
F = 210°C
CCM gel de silice 60 F 254 Merck
CH₂Cl₂ - MeOH : 90-10 Rf = 0,68.

### Exemple 2 : Trans 2-[4-[(2,3-dihydro-benzo[1,4]dioxin-2-ylméthyl)-amino]-cyclo-hexyl]-4-méthyl-2H-[1,2,4]triazine-3,5-dione hemifumarate (2).

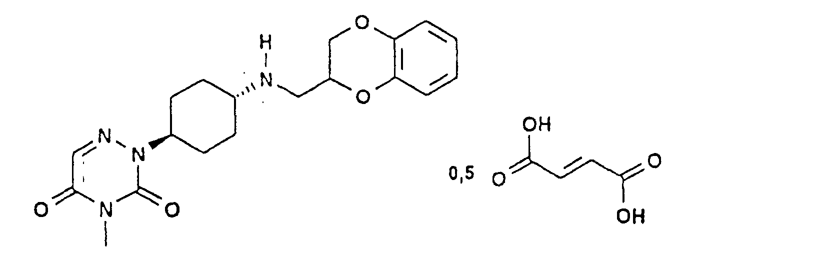

Ce composé est préparé selon le procédé décrit dans l'exemple 1 en utilisant au stade f un mélange cis et trans (30/70) de 4-[(2,3-dihydro-benzo[1,4]dioxin-2-ylméthyl)-amino]-cyclohexanol (préparé selon le procédé décrit dans l'exemple 1 en utilisant NaBH₄ dans l'EtOH au stade e), puis salifié par l'acide fumarique dans le méthanol.
F = 238°C
CCM gel de silice 60 F 254 Merck
CH₂Cl₂ - MeOH : 90-10 Rf = 0,63.

### Exemple 3 : Cis 4-méthyl-2-[4-(4-pyrimidin-2-yl-pipérazin-1-yl)-cyclohexyl]-2H-[1,2,4]triazine-3,5-dione(3).

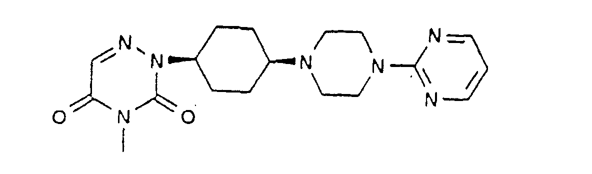

Ce composé est préparé selon le procédé décrit dans l'exemple 2 en utilisant un mélange cis et trans (30/70) de 4-(4-pyrimidin-2-yl-pipérazin-1-yl)-cyclohexanol (préparé selon le procédé décrit dans l'exemple 1 en utilisant NaBH₄ dans l'EtOH au stade e).
F = 166°C
CCM gel de silice 60 F 254 Merck
CH₂Cl₂ - MeOH : 90 - 10 Rf = 0,46.

### Exemple 4 : Trans 4-méthyl-2-[4-(4-pyrimidin-2-yl-pipérazin-1-yl)-cyclohexyl]-2H-[1,2,4]triazine-3,5-dione (4).

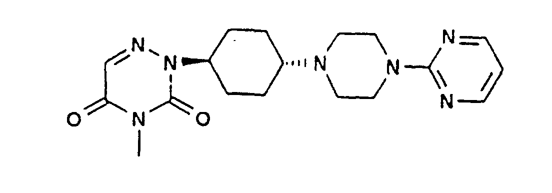

Ce composé est préparé selon le procédé décrit dans l'exemple 3.
F = 222°C
CCM gel de silice 60 F 254 Merck
CH₂Cl₂ - MeOH : 90 - 10 Rf = 0,31.

### Exemple 5 : Trans 4-méthyl-2-[4-[4-(4,6-diméthyl-pyrimidin-2-yl)-pipérazin-1-yl]-cyclohexyl]-2H-[1,2,4]triazine-3,5-dione (5).

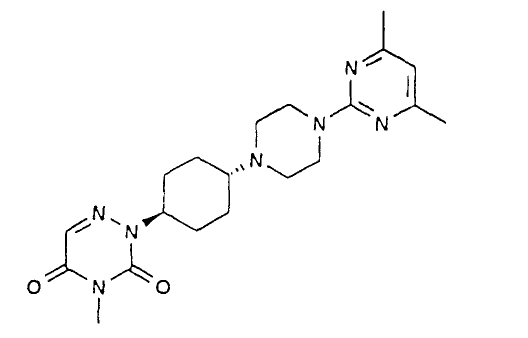

### a) Cis 4-[4-(4,6-diméthyl-pyrimidin-2-yl)-pipérazin-1-yl]-cyclohexanol (5a).

Le L-S-sélectride (solution 1M dans le THF ; 22,6 ml ; 22,6 mmol) est placé à -78°C sous atmosphère inerte.

La 4-[4-(4,6-diméthyl-pyrimidin-2yl)-pipérazin-1-yl]cyclohexanone (préparée selon l'exemple 1 à partir de (4,6-diméthyl-2-pipérazin-1-yl)pyrimidine) (5,9 g ; 20,4 mmol) diluée dans 25 ml de THF à 0°C est coulée goutte à goutte. Ce mélange est agité 2 h à -78°C puis la température est portée à l'ambiante.

Le milieu réactionnel est hydrolysé avec H₂O puis extrait au dichlorométhane. Les phases organiques sont séchées sur sulfate de magnésium puis concentrées à sec. Le résidu isolé est purifié par chromatographie flash sur silice (éluent CH₂Cl₂ - MeOH - NH₄OH : 90 - 9 - 1). On isole 3,8 g d'alcool **5a**.
CCM gel de silice 60 F 254 Merck
CH₂Cl₂ - MeOH - NH₄OH : 90 - 9 - 1 Rf = 0,50.

### b) Trans 4-méthyl-2-[4-[4-(4,6-diméthyl-pyrimidin-2-yl)-pipérazin-1-yl]-cyclohexyl]-2H-[1,2,4]triazine-3,5-dione (5)

Ce composé est préparé selon le procédé décrit dans l'exemple 1 en utilisant au stade f l'alcool **5a**.
F = 255°C
CCM gel de silice 60 F 254 Merck
CH₂Cl₂ - MeOH - NH₄OH : 90 - 9 - 1 Rf = 0,60.

### Exemple 6 : Trans 4-méthyl-2-[4-[4-(4-méthyl-pyrimidin-2-yl)-pipérazin-1-yl]-cyclo-hexyl]-2H-[1,2,4]triazine-3,5-dione(6).

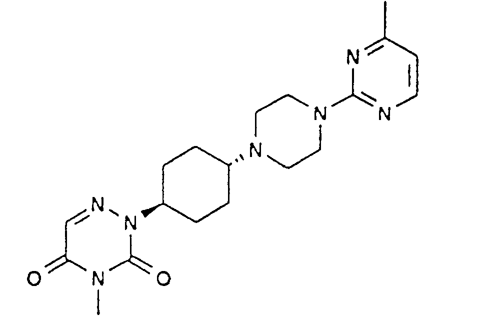

Ce composé est préparé selon le procédé décrit dans l'exemple 1 en utilisant au stade f le cis 4-[4-(4-méthyl-pyrimidin-2-yl)-pipérazin-1-yl]-cyclohexanol (préparé selon le procédé décrit dans l'exemple 5 au stade a).
F = 210°C
CCM gel de silice 60 F 254 Merck
CH₂Cl₂ - MeOH : 90 - 10 Rf = 0,41.

### Exemple 7 : Trans 2-[4-[4-(4-chloro-pyrimidin-2-yl)-pipérazin-1-yl)-cyclohexyl]-4-méthyl-2H-[1,2,4]triazine-3,5-dione (7).

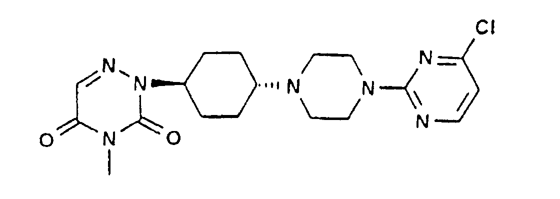

### a) Cis 4-pipérazin-1-yl-cyclohexanol (7a).

Le cis 4-(4-benzyl-pipérazin-1-yl)-cyclohexanol (préparé selon le procédé décrit dans l'exemple 5 en utilisant la 4-(4-benzyl-pipérazin-1-yl)-cyclohexanone au stade a) (11,6 g ; 30,3 mmol) est placé dans 80 ml de dichlorométhane à 0°C. Le chloroformiate d'α-chloroéthyle (9,9 ml ; 90,9 mmol) est coulé goutte à goutte et le mélange est agité 0,5 h à 0°C.

Le milieu réactionnel est concentré à sec puis repris par 80 ml de méthanol. Après avoir placé ce mélange à reflux pendant 45 mn, la solution est concentrée à sec. Le résidu obtenu est repris par H₂O (pH = 11) et extrait au dichlorométhane. Les phases organiques sont séchées (MgSO₄) puis concentrées à sec. Après purification par chromatographie flash sur silice (éluent CH₂Cl₃ - MeOH - NH₄OH : 80 - 18 - 2), puis cristallisation dans l'éther, on isole 1,7 g de solide brun.
CCM gel de silice 60 F 254 Merck
CH₂Cl₂ - MeOH - NH₄OH : 80 - 18 - 2 Rf = 0,30.

### b) Trans 2-[4-[4-(4-chloro-pyrimidin-2-yl)-pipérazin-1-yl]-cyclohexyl]-4-méthyl-2H-[1,2,4]triazine-3,5-dione (7).

Le composé 7a (1,5 g ; 5,1 mmol) est placé dans 40 ml de toluène en présence de 2,4-dichloropyrimidine (0,84 g ; 5,6 mmol) et de triéthylamine (0,78 ml ; 5,6 mmol).

Après avoir chauffé ce mélange à reflux pendant 1,5 h, le solvant est évaporé sous vide. Le résidu est repris avec H₂O puis extrait avec CH₂Cl₂. Après séchage des phases organiques (MgSO₄) et concentration à sec, l'huile claire obtenue est purifiée par chromatographie flash sur silice (éluent CH₂Cl₂ - MeOH : 95 - 5). On isole 0,4 g de solide blanc.
F = 201°C
CCM gel de silice 60 F 254 Merck
CH₂Cl₂ - MeOH : 95 - 5 Rf = 0,22.

### Exemple 8 : Trans 2-[4-[4-(5-fluoro-pyrimidin-2-yl)-pipérazin-1-yl]-cyclohexyl]-4-méthyl-2H-[1,2,4]triazine-3,5-dione (8).

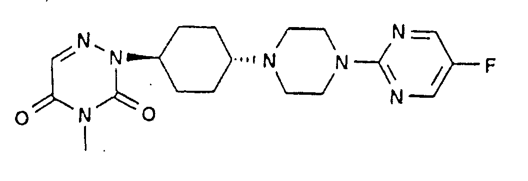

Ce composé est préparé selon l'exemple 7 en utilisant au stade b la 2-chloro-5-fluoropyrimidine.
F = 220°C
CCM gel de silice 60 F 254 Merck
CH₂Cl₂ - MeOH : 90 - 10 Rf = 0,35.

### Exemple 9 : Trans 2-méthyl-5-[4-(4-pyrimidin-2-yl-pipérazin-1-yl)-cyclohexyloxy] -2H-[1,2,4]triazin-3-one (9).

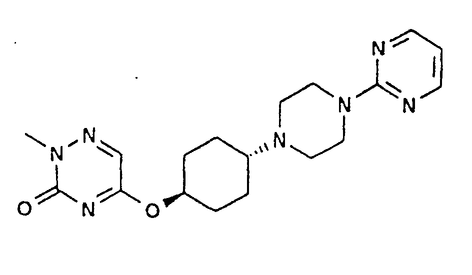

### a) 5-Thioxo-4,5 dihydro-2H-[1,2,4]triazin-3-one (9a).

Dans une solution de 2*H*-[1,2,4]triazin-3,5 dione (50 g ; 442 mmol) dans 400 ml de pyridine sont ajoutés 98,3 g (243 mmol) de réactif de Lawesson. Le mélange est porté à reflux pendant 4 h. Après évaporation du solvant sous pression réduite, le résidu obtenu est repris par 400 ml d'eau. Le précipité brun qui se forme est isolé par filtration. Ces cristaux sont repris avec H₂O et extraits à l'acétate d'éthyle. Après séchage (MgSO₄) et concentration à sec des phases organiques, on obtient des cristaux jaunes.

Le retraitement de l'eau (400 ml) par extraction à l'acétate d'éthyle permet d'isoler une nouvelle fraction de solide. Au total, après séchage, 60 g de cristaux jaunes sont obtenus,
F = 239°C
CCM gel de silice 60 F 254 Merck
CH₂Cl₂ - MeOH : 90 - 10 Rf = 0,4.

### b) 5-Méthylsulfanyl-2H-[1,2,4]triazin-3-one (9b).

Le composé **9a** (30 g ; 232 mmol) et CH₃I (15,9 ml ; 255 mmol) sont placés dans 300 ml d'eau. 18,6 g de NaOH (465 mmol) sont additionnés et le mélange est agité 1 h à température ambiante. Le milieu réactionnel, refroidi sur lit de glace, est neutralisé à l'aide de 27 ml d'acide acétique puis extrait au dichlorométhane. Les phases organiques sont séchées (MgSO₄) puis concentrées à sec. Après recristallisation dans l'éther, on isole 29,9 g de composé **9b.**
F = 171°C
CCM gel de silice 60 F 254 Merck
CH₂Cl₂ - MeOH : 90 - 10 Rf = 0,5.

### c) 2-Méthyl-5-méthylsulfanyl-2H-[1,2,4]triazin-3-one (9c).

Une suspension de NaH (60 % dans l'huile de paraffine, 4,4 g ; 110 mmol) dans 50 ml de DMF placée sous azote est refroidie à 0°C sur lit de glace. Le composé **9b**
(15,9 g ; 111 mmol) dilué dans 100 ml de DMF est coulé goutte à goutte dans cette suspension. Le mélange est ensuite agité 1 h à température ambiante.

Après concentration à sec du milieu réactionnel, le résidu est repris par H₂O et extrait avec CH₂Cl₂. Les phases organiques sont séchées sur MgSO₄ puis évaporées sous pression réduite.

Après cristallisation dans EtOH/éther isopropylique, puis séchage, on isole 13,9 g de produit **9c** sous forme de cristaux beiges.
F = 106°C
CCM gel de silice 60 F 254 Merck
CH₂Cl₂-MeOH : 95-5 Rf = 0,52.

### d) Trans: 2-méthyl-5-[4-(4-pyrimidin-2-yl-pipérazin-1-yl)-cyclohexyloxyl-2H-[1,2,4]-triazin-3-one (9).

NaH (60 % dans la paraffine ; 0,56 g ; 14 mmol) est placé en suspension dans 20 ml de dioxane sous atmosphère inerte à 0°C.

Le trans 4-(4-pyrimidin-2-yl-pipérazin-1-yl)-cyclohexanol (préparé selon le procédé décrit dans l'exemple 1 en utilisant au stade e NaBH₄ dans l'éthanol) (4,0 g ; 15,4 mmol) dilué dans 20 ml de dioxane est alors additionné. Le mélange est agité pendant que la température remonte à l'ambiante.

Après avoir replacé le milieu réactionnel sur lit de glace, le composé **9c** (2,2 g ; 14 mmol) dilué dans 15 ml de dioxane est ajouté et le milieu réactionnel est agité 1 h à 0°C.

Après concentration à sec, le résidu obtenu est repris par l'eau puis extrait au dichlorométhane. Les phases organiques sont séchées sur MgSO₄ puis concentrées à sec. L'huile isolée est purifiée par chromatographie flash sur silice (éluent CH₂Cl₂-MeOH-NH₄OH : 90 - 9 - 1). Après recristallisation dans le méthanol, on isole 2 g de précipité blanc.
F = 200°C
CCM gel de silice 60 F 254 Merck
CH₂Cl₂ - MeOH - NH₄OH : 90 - 9 - 1 Rf = 0,35.

### Exemple 10 : Trans 2-méthyl-5-[4-[4-(4-méthyl-pyrimidin-2-yl)-pipérazin-1-yl]-cy-clohexyloxy]-2H-[1,2,4]triazin-3-one (10).

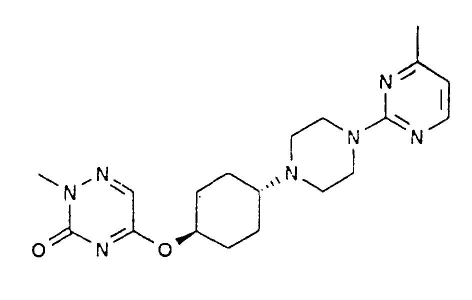

### a) Trans 4-Dipérazin-1-yl-cyclohexanol (10a)

Le trans 4-(4-benzyl-pipérazin-1-yl)-cyclohexanol (préparé à partir de la benzylpipérazine selon le procédé décrit dans l'exemple 1 en utilisant au stade e NaBH₄ dans l'éthanol) (15 g ; 54,6 mmol) est placé en présence de palladium sur charbon (1 g à 10 %) dans 150 ml d'éthanol sous 4 bars d'hydrogène. Après 72 h d'agitation, le catalyseur est filtré sur célite et le filtrat concentré à sec. On isole 9,6 g d'huile claire.
CCM gel de silice 60 F 254 Merck
CH₂Cl₂ - MeOH - NH₄OH : 80 - 18 - 2 Rf = 0,42.

### b) Trans 4-[4-(4-méthyl-pyrimidin-2-yl)-pipérazin-1-yl]-cyclohexanol (10b).

Le composé **10a** (4,3 g ; 23,3 mmol) est placé en présence de 2-chloro,4-méthyl-pyrimidine (3 g ; 23,3 mmol) et de triéthylamine (4,9 ml ; 35,1 mmol) dans 100 ml de toluène puis ce mélange est chauffé à reflux pendant 16 h.

Après évaporation sous vide du solvant, le résidu et repris par H₂O basique (pH = 11) et extrait au dichlorométhane. Les phases organiques sont séchées (MgSO₄) puis concentrées à sec. Le résidu isolé est purifié par chromatographie flash sur silice (éluent CH₂Cl₂ - MeOH - NH₄OH : 80 - 18 - 2) et on isole 4 g de composé **10 b**.
CCM gel de silice 60 F 254 Merck
CH₂Cl₂ - MeOH - NH₄OH : 80 - 18 - 2 Rf = 0,77.

### c) Trans 2-méthyl-5-[4-[4-(4-méthyl-pyrimidin-2-yl)-pipérazin-1-yl]-cyclohexyl-oxy]-2H-[1,2,4]triazin-3-one (10).

Ce composé est préparé selon le procédé décrit dans l'exemple 9 en utilisant l'intermédiaire **10b** au stade d.
F = 189°C,
CCM gel de silice 60 F 254 Merck
CH₂Cl₂ - MeOH : 90 - 10 Rf = 0,44

### Exemple 11 : Trans 2-méthyl-5-[4-[4-(4,6-diméthyl-pyrimidin-2-yl)-pipérazin-1-yl]-cyclohexyloxyl-2H-[1,2,4]triazin-3-one (11).

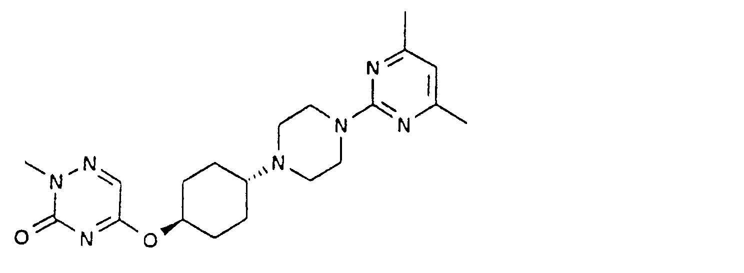

Ce composé est préparé selon le procédé décrit dans l'exemple 10 en utilisant la 2-chloro, 4,6-diméthylpyrimidine au stade b.
F = 211°C
CCM gel de silice 60 F 254 Merck
CH₂Cl₂ - MeOH : 90 - 10 Rf = 0,54.

### Exemple 12 : Trans 2-méthyl-5-[4-[4-(4-chloro-pyrimidin-2-yl)-pipérazin-1-yl]-cyclohexyloxy]-2H-[1,2,4]triazin-3-one (12).

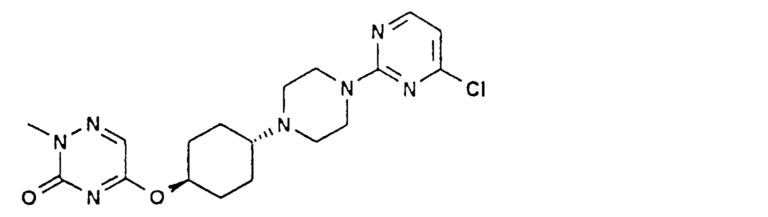

Ce composé ;est préparé selon le procédé décrit dans l'exemple 10 en utilisant la 2, 4-dichloropyrimidine au stade b.
F = 214°C
CCM gel de silice 60 F 254 Merck
CH₂Cl₂ - MeOH : 90-10 Rf = 0,57.

### Exemple 13 : Trans 2-méthyl-5-[4-[4-(4-méthoxy-pyrimidin-2-yl)-pipérazin-1-yl]-cyclohexyloxyl-2H-[1,2,4]triazin-3-one (13).

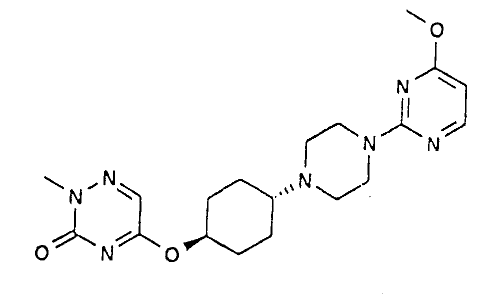

Ce composé est préparé selon le procédé décrit dans l'exemple 10 en utilisant la 2-chloro, 4-méthoxypyrimidine au stade b.
F = 196°C
CCM gel de silice 60 F 254 Merck
CH₂Cl₂ - MeOH : 90 - 10 Rf = 0,43.

### Exemple 14 : Trans 2-méthyl-5-[4-[4-(4-trifluorométhylpyrimidin-2-yl)-pipérazin-1-yl]-cyclohexyloxyl-2H-[1,2,4]triazin-3-one (14).

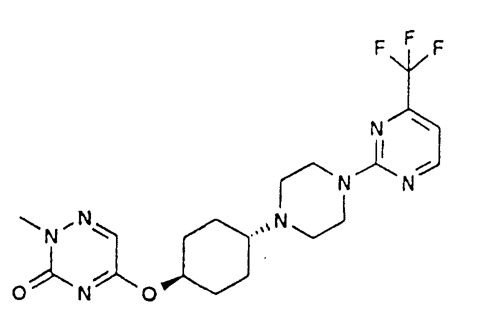

Ce composé est préparé selon le procédé décrit dans l'exemple 10 en utilisant la 2-chloro, 3-trifluorométhylpyrimidine au stade b.
F = 184°C
CCM gel de silice 60 F 254 Merck
CH₂Cl₂ - MeOH : 90 - 10 Rf = 0,73.

### Exemple 15 : Trans 2-methyl-5-[4-[4-(5-fluoro-pyrimidin-2-yl)-pipérazin-1-yl]-cyclohexyloxy]-2H-[1,2,4]triazin-3-one (15).

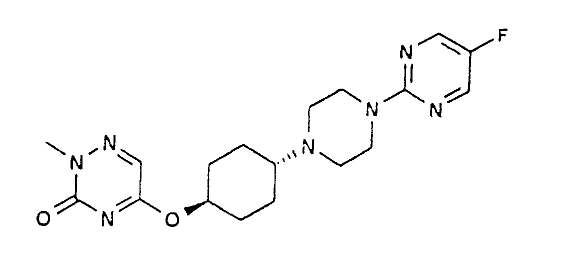

Ce composé est préparé selon le procédé décrit dans l'exemple 10 en utilisant la 2-chloro,5-fluoropyrimidine au stade b.
F = 209°C
CCM gel de silice 60 F 254 Merck
CH₂Cl₂ - MeOH : 90 - 10 Rf = 0,61.

### Exemple 16 : Trans 5-[4-[(2,3-dihydro-benzo[1,4]dioxin-2-ylméthyl)-amino]-cyclo-hexyloxyl-2-méthyl-2H-[1,2,4]triazin-3-one (16).

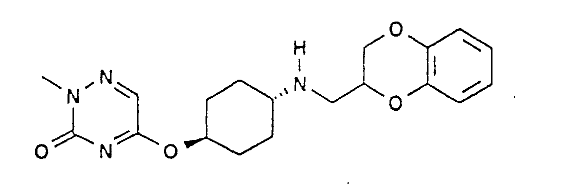

Ce composé est préparé selon le procédé décrit dans l'exemple 9 en utilisant le trans 4-[(2,3-dihydro-benzo[1,4]dioxin-2-ylméthyl)-amino]-cyclohexanol (préparé selon le procédé décrit dans l'exemple 1 en utilisant au stade e NaBH₄ dans l'éthanol) au stade d.
F = 114°C
CCM gel de silice 60 F 254 Merck
CH₂Cl₂ - MeOH : 90 - 10 Rf = 0,61.

### Exemple 17 : Trans 6-{4-[4-(3-chloro-phényl)-pipérazin-1-yl]-cyclohexylamino}-2,4-diméthyl-2H-[1,2,4]triazine-3,5-dione (17). (ne fait pas partie de l'invention)

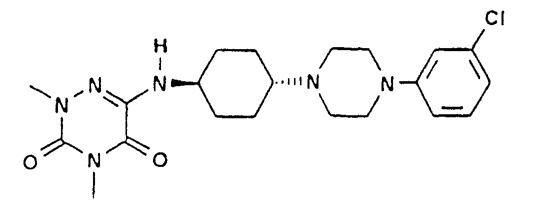

### a) 2,4-Diméthyl-2H-[1,2,4]triazino-3,5-dione (17a).

NaH (60 dans la paraffine ; 8,8 g ; 220 mmol) est placé en suspension dans 100 ml de DMF sous atmosphère inerte. Une solution de 2*H*-[1,2,4]triazin-3,5 dione (25 g ; 220 mmol) est ajoutée goutte à goutte puis ce mélange est agité 0,5 h à température ambiante. CH₃I (27,4 ml ; 440 mmol) est additionné puis l'agitation est maintenue pendant une nuit.

Le solvant est concentré à sec sous vide puis le résidu est repris par 300 ml de DMF, auxquels sont additionnés, sous atmosphère inerte, 8,8 g de NaH (60 % dans la paraffine ; 220 mmol).

Après 4 h d'agitation, CH₃l est additionné (27,4 ml ; 440 mmol) et le mélange est agité une nuit à température ambiante.

Le milieu réactionnel est concentré à sec sous vide et le résidu isolé est repris par une solution aqueuse saturée de NaCl et extrait à l'acétate d'éthyle. Les phases organiques sont séchées sur Na₂SO₄ puis évaporées sous vide. Après cristallisation et lavage à l'eau, on obtient 16,4 g de composé **17a**.
F = 64°C
CCM gel de silice 60 F 254 Merck
CH₂Cl₂ - AcOEt : 70-30 Rf = 0,53.

### b) 6-Bromo-2,4-diméthyl-2H-[1,2,4]triazine-3,5-dione (17 b).

Le composé **17a** (13,3 g ; 94,1 mmol) est placé dans 100 ml d'eau en présence de brome (18 ml ; 351 mmol) puis ce mélange est chauffé à 60°C pendant 12 h.

Après concentration à sec, le résidu obtenu est repris par H₂O puis extrait à l'acétate d'éthyle. Les phases organiques sont séchées sur MgSO₄ puis évaporées sous vide. Après recristallisation dans l'éther, on isole 7,8 g de composé **17b.**
F = 104°C
CCM gel de silice 60 F 254 Merck
CH₂Cl₂ - AcOEt : 70 - 30 Rf = 0,73.

### c) 4-[4-(3-Chloro-phényl)-pipérazin-1-yl]-cyclohexanone oxime (17c).

Le composé **1d** (15,3 g ; 52,3 mmol) est placé en présence de chlorhydrate d'hydroxylamine (5,9 g ; 83,7 mmol) et de soude (14,5 g ; 130,7 mmol) dans un mélange de 143 ml d'EtOH et 7 ml d'eau.

Le mélange est agité 1 h à température ambiante puis 70 ml d'eau sont additionnés. Le milieu réactionnel est extrait à l'acétate d'éthyle.

Les phases organiques sont séchées sur MgSO₄ puis concentrées à sec. On isole 15,3 g de solide orange utilisé sans autre purification pour l'étape suivante.
CCM gel de silice 60 F 254 Merck
CH₂Cl₂ - MeOH : 95 - 5 Rf = 0,12.

### d) Cis et trans 4-[4-(3-chloro-phényl)-pipérazin-1-yl]-cyclohexylamine (17d).

LiAlH₄ (8,4 g ; 221 mmol) est placé en suspension dans 219 ml de THF sous atmosphère inerte. Le composé **17c** (20,8 g ; 67,6 mmol) dilué dans 227 ml de THF est additionné goutte à goutte puis le mélange est porté à reflux pendant 2 h.

Le milieu réactionnel est neutralisé à l'aide de 32 ml d'eau puis séché avec MgSO₄. Après filtration sur fritté, le filtrat est concentré à sec. L'huile orange isolée est purifiée par chromatographie flash sur silice (éluent CH₂Cl₂ - MeOH - NH₄OH : 80 - 18 - 2). On récupère 3,4 g de composé **17d** cis et 3,7 g de composé **17d** trans.
CCM gel de silice 60 F 254 Merck
CH₂Cl₂ - MeOH - NH₄OH : 80 - 18 - 2 Rf_{cis} = 0,43 Rfₜᵣₐₙₛ = 0,31.

### e) Trans 6-{4-[4-(3-chloro-phényl)-pipérazin-1-yl]-cyclohexylamino}-2,4-diméthyl-2H-[1,2,4]triazine-3,5-dione (17).

Le composé **17d** trans (3,7 g ; 12,4 mmol) est placé en présence d'intermédiaire **17b** dans 45 ml de nBuOH et de triéthylamine (3,6 ml ; 25,8 mmol). Ce mélange est porté à reflux pendant 36 h puis le solvant est concentré à sec sous vide. Le résidu brun obtenu est repris par H₂O et extrait à l'acétate d'éthyle. Les phases organiques sont séchées sur MgSO₄ puis évaporées sous vide. Après purification par chromatographie flash sur silice (éluent CH₂Cl₂ - MeOH : 95 - 5), et décoloration au noir animal dans l'éthanol, on isole un précipité blanc (0,5 g).
F = 178°C
CCM gel de silice 60 F 254 Merck
CH₂Cl₂ - MeOH : 90 - 10 Rf = 0,67.

### Exemple 18 : Cis 6-{4-[4-(3-chloro-phényl)-pipérazin-1-yl]-cyclohexylamino}-2,4-diméthyl-2H-[1,2,4]triazine-3,5-dione (18). (ne fait pas partie de l'invention)

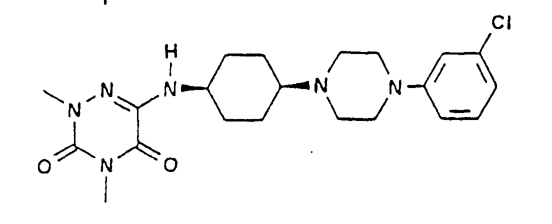

Ce composé est préparé selon le procédé décrit dans l'exemple 17 en utilisant au stade e l'intermédiaire **17d** cis.

### Exemple 19 : Trans 6-({4-[4-(3-chloro-phényl)-pipérazin-1-yl]-cyclohexyl}-méthyl-amino)-2,4-diméthyl-2H-[1,2,4]triazine-3,5-dione (19).

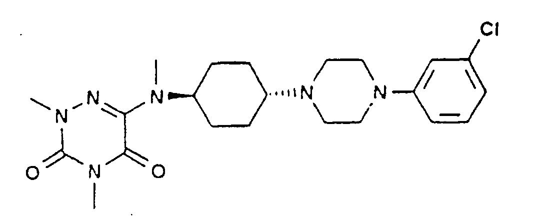

Le composé **17** (0,5 g ; 1,2 mmol) est placé en présence de NaBH₄ (0,2 g ; 5,3 mmol) et de paraformaldéhyde (0,3 g ; 11,3 mmol) dans 14 ml de THF sous atmosphère inerte. L'acide trifluoroacétique (7 ml ; 90,8 mmol) est additionné goutte à goutte puis ce mélange est agité 20 h à température ambiante.

Le milieu réactionnel est additionné à une solution contenant 19 ml de NaOH 25 % et 19 ml de solution aqueuse saturée en NaCl.

Après extraction au dichlorométhane, les phases organiques sont séchées sur MgSO₄ puis concentrées à sec. Le résidu obtenu est purifié par chromatographie flash sur silice (éluent CH₂Cl₂ - MeOH : 95 - 5). Après cristallisation dans l'éthanol, on isole 0,4 g de solide blanc.
F = 179°C.
CCM gel de silice 60 F 254 Merck
CH₂Cl₂ - MeOH : 95 - 5 Rf = 0,38.

### Exemple 20 : Cis 6-({4-[4-(3-chloro-phényl)-pipérazin-1-yl]-cyclohexyl}-méthyl-amino)-2,4-diméthyl-2H-[1,2,4]triazine-3,5-dione (20).

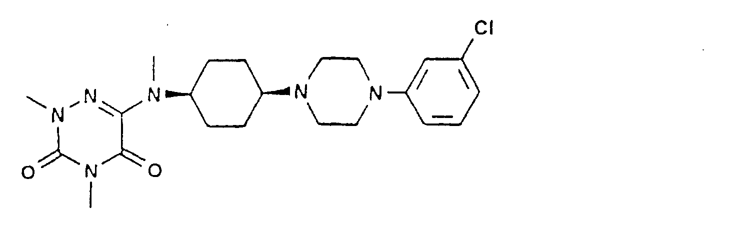

Ce composé est préparé selon le procédé décrit dans l'exemple 19 à partir de l'intermédiaire **18**.
F = 124°C
CCM gel de silice 60 F 254 Merck
CH₂Cl₂ - MeOH : 95 - 5 Rf = 0,58.

### Exemple 21 : Trans 2,4-diméthyl-6-[4-(4-pyrimidin-2-yl-pipérazin-1-yl)-cyclo-hexyl-amino]-2H-[1,2,4]triazine-3,5-dione (21). (ne fait pas partie de l'invention)

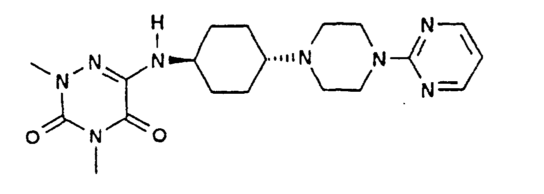

Ce composé est préparé selon le procédé décrit dans l'exemple 17 en utilisant la trans 4-[4-pyrimidin-2-yl-pipérazin-1-yl]-cyclohexylamine au stade e.
F = 173°C
CCM gel de silice 60 F 254 Merck
CH₂Cl₂ - MeOH : 90 - 10 Rf = 0,46.

### Exemple 22 : Cis 2,4-diméthyl-6-[4-(4-pyrimidin-2-yl-pipérazin-1-yl)-cyclohexyl-amino]-2H-[1,2,4]triazino-3,5-diono (22). (ne fait pas partie de l'invention)

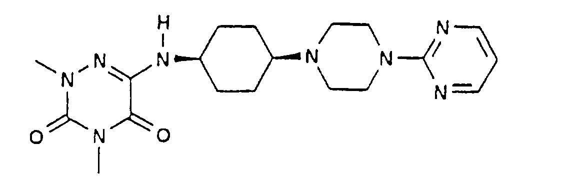

Ce composé est préparé selon le procédé décrit dans l'exemple 17 en utilisant la cis 4-[4-pyrimidin-2-yl-pipérazin-1-yl]-cyclohexylamine au stade e.
F = 170°C
CCM gel de silice 60 F 254 Merck
CH₂Cl₂ - MeOH : 90 - 10 Rf = 0,47.

Les composés de l'invention ont été soumis à des essais pharmacologiques qui ont mis en évidence leur intérêt comme substances actives en thérapeutique.

### Liaison aux récepteurs 5-HT_{1A}, D₂ dopaminergiques et α1-adrénergiques :

Des cerveaux de rats mâles Sprague Dawley 180-200 g [Ico : OFA SD (I.O.P.S. Caw) ; Iffa Credo, France], maintenus à - 70°C ont été utilisés dans toutes les études.

L'affinité des produits pour les différents récepteurs a été déterminée par déplacement de ligands radioactifs dans les conditions résumées dans le tableau 1.

La réaction est stoppée par filtration rapide, sous vide, sur filtres whatman GF/B, les tubes sont rincés avec 2 x 5 ml de tampon Tris-HCl 50 mM, pH 7,4 à 25°C. La radioactivité recueillie sur le filtre est analysée en scintillation liquide après ajout de 4 ml de liquide scintillant (Emulsifier Safe, Packard). Toutes les expériences sont réalisées en triple.

Les constantes d'inhibition (Ki) des produits sont estimées à partir des expérimentations de déplacement en utilisant le programme de régression non-linéaire RADLIG version 4 de EBDA (equilibrium binding data analysis) (Biosoft, Cambridge, UK; McPherson, 1985).

Les valeurs de pKi (-log Ki) sont données sous forme de moyenne ± SEM d'au moins 3 expérimentations (tableau 2).

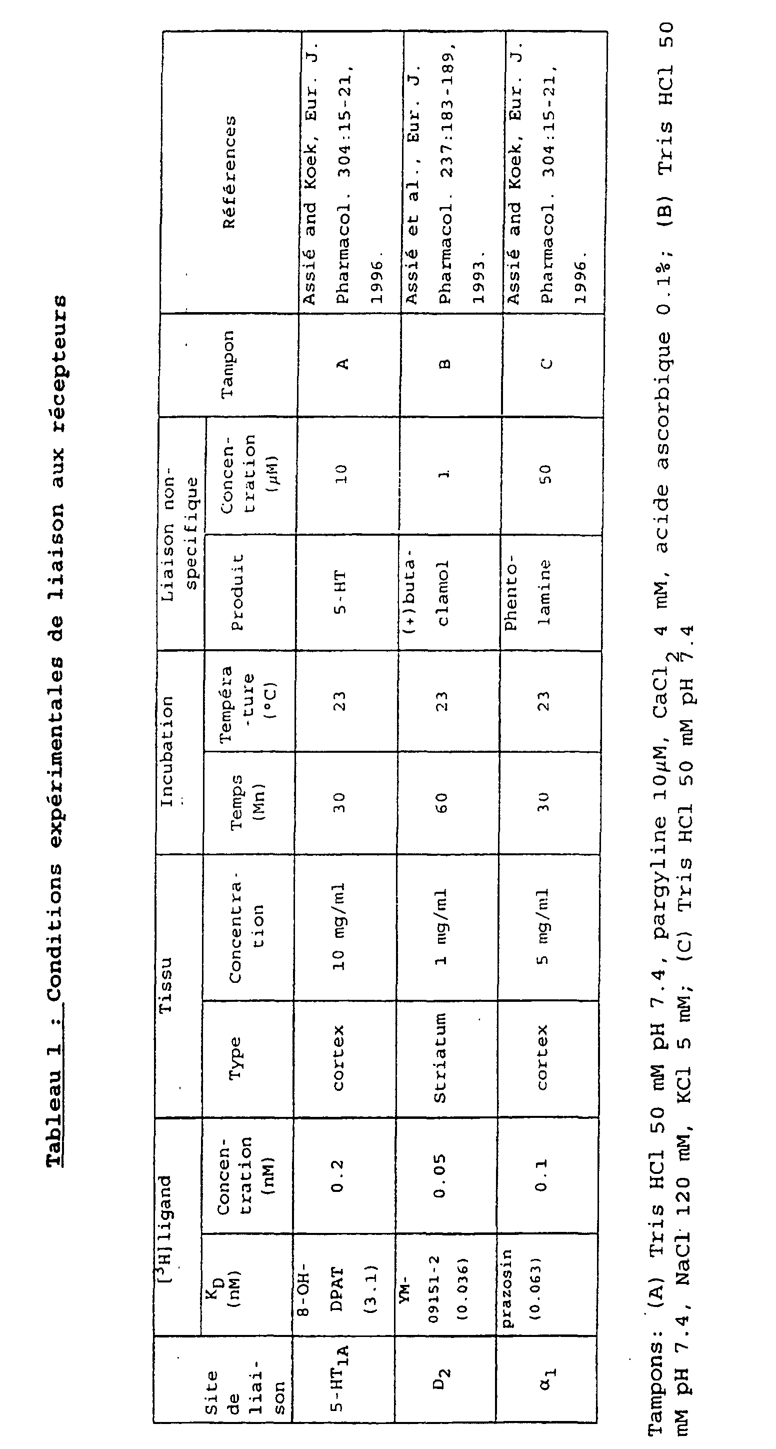

**Tableau 2**

| Composé n° | pKi | | |
|---|---|---|---|
| | 5HT_{1A} | α₁ | D₂ |
| 4 | 8,43 | < 5,0 | < 5,0 |
| 5 | 9,22 | 5,89 | < 5,0 |
| 6 | 9,09 | 6,10 | < 5,0 |
| 9 | 9,15 | < 5,0 | < 5,0 |
| 10 | 9,60 | 5,95 | < 5,0 |
| 11 | 9,60 | 5,72 | < 5,0 |
| 12 | 9,54 | 6,21 | < 5,0 |
| 13 | 9,28 | 6,65 | < 5,0 |
| 16 | 9,78 | - | - |
| 17 | 9,40 | 7,74 | 6,22 |
| 19 | 9,71 | 7,86 | < 5,0 |
| Buspirone | 7,65 | 6,19 | 7,49 |
| Flesinoxan | 8,91 | 6,50 | 7,05 |

### Syndrome sérotoninergique :

L'activité centrale des composés de l'invention a été évaluée par leur capacité à provoquer le syndrome 5-HT qui est caractérisé par :
- une flexion et une extension alternée des pattes avant (reciprocal fore-paw treading : FPT)
- la rétraction de la lèvre inférieure (lower lip retraction : LLR)
- une position ou la surface ventrale de l'animal est en contact avec le sol et les pattes arrières étendues (flat body posture : FBP).

Les expériences de l'évaluation du syndrome 5-HT sont réalisées chez le rat mâle (Sprague-Dawley) selon la technique décrite par F.C. COLPAERT et al. (Drug. Dev. Res., 26, 21-48; 1992) et M.S. KLEVEN et al. (J.P.E.T., 282 : 747-759, 1997).

Le tableau 3 donne, à titre d'exemple, les doses actives (ED₅₀) pour certains dérivés de l'invention par rapport aux produits de référence tels que Buspirone et Flesinoxan.

**Tableau 3**

| Syndrome 5-HT | | | |
|---|---|---|---|
| Composé n° | ED₅₀ mg/kg po | | |
| | FBP | LLR | FPT |
| 4 | 1,25 | 0,31 | 1,25 |
| 5 | 0,08 | < 0,04 | 0,08 |
| 6 | 0,08 | 0,08 | 0,08 |
| 9 | 0,08 | 0,08 | 0,31 |
| 10 | 0,08 | 0,02 | 0,08 |
| 11 | 0,02 | 0,02 | 0,08 |
| 12 | 0,08 | 0,08 | 0,08 |
| 13 | 0,31 | 0,08 | 0,31 |
| 14 | 0,31 | 0,08 | 0,31 |
| 15 | 1,25 | 0,31 | > 2,5 |
| 16 | 0,31 | 0,08 | > 2,5 |
| Buspirone | 20 | 2,5 | > 40 |
| Flesinoxan | 1,25 | 1,25 | 5 |

### Activité antidépressive : Test de la nage forcée :

Les composés de l'invention sont testés selon la procédure décrite par R. PORSOLT et al. (Eur. J. Pharmacol. 47 : 379-391 ; 1978).

Les doses actives (ED₅₀) sont calculées pour chaque composé on fonction du pourcentage d'animaux présentant une diminution significative par rapport aux animaux témoins (p < 0.05) du temps d'immobilité (tableau 4).

**Tableau 4**

| Composé n° | ED₅₀ mg/kg po |
|---|---|
| 4 | 1,25 |
| 5 | 0,31 |
| 9 | 0,31 |
| 10 | 0,08 |
| 11 | 0,08 |
| 12 | 0,74 |
| Buspirone | > 160 |
| Flesinoxan | 1,25 |

Les résultats des différents essais montrent que les composés de formule générale I possèdent in vitro, une haute affinité pour les récepteurs sérotoninergiques de type 5HT_{1A} et une bonne sélectivité vis-à-vis des récepteurs α₁ et D₂. In vivo, ils montrent une activité agoniste vis-à-vis des récepteurs 5HT_{1A} et sont puissamment actifs sur des modèles comportementaux tel que le test de la nage forcée.

Les composés de l'invention peuvent donc être utiles dans le traitement de l'anxiété, la dépression, la douleur, la neurodégénérescence, la schizophrénie, la maladie d'Alzheimer, les troubles du sommeil, pour la régularisation de prise de nourriture, pour la régularisation de la sécrétion gastrique, et pour le traitement des désordres vasculaires, cardiovasculaires et cérébro-vasculaires tels que l'hypertension ou la migraine.

Les préparations pharmaceutiques contenant comme principe actif des composés de formule générale I peuvent être mises en forme pour l'administration par voie orale, rectale ou parentérale, par exemple sous la forme de capsules, comprimés, granulés, gélules, solutés liquides, sirops ou suspensions buvables, et contenir les excipients appropriés.

Il est également possible d'y associer d'autres principes actifs pharmaceutiquement et thérapeutiquement acceptables.

## Revendications

1. Dérivés cyclohexaniques difonctionnalisés correspondant à la formule générale I 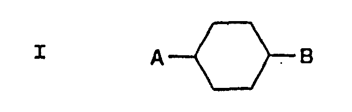 dans laquelle
- A représente un groupement de formule 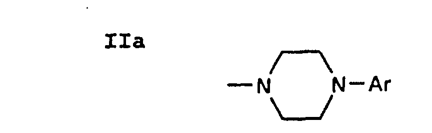 dans lequel Ar représente lui-même une structure de type aromatique choisie parmi phényle ou pyrimidinyle, éventuellement substituée par un ou plusieurs groupements alcoyle en C₁₋₃, alcoxy en C₁-C₃, trifluorométhyle ou halogène 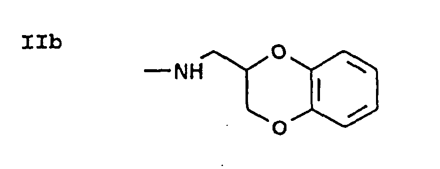
- B représente un groupement hétérocyclique de formules
. 3,5-dioxo-(*2H*,*4H*)-1,2,4 triazine substitué en position 2 (IIIa) 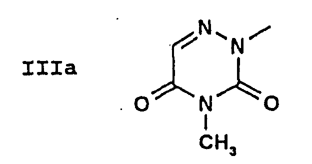
. 3-oxo-(2H)-1,2,4 triazine substitué en position 5 (IIIb) 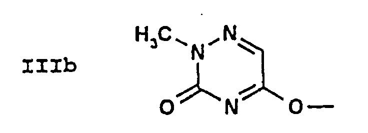 ou bien :
. 3,5-dioxo-6-amino-(2*H*, 4*H*)-1,2,4-triazine (IIIc) 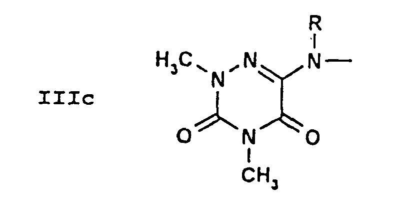
dans lequel R représente un groupement alcoyle en C₁-C₃
ainsi que les sels des composés de formule générale I avec les acides pharmaceutiquement acceptables,
les composés de formule I pouvant se présenter sous la forme de différents isomères « cis » et « trans » ainsi que de différents énantiomères des composés possédant des carbones asymétriques.

2. Composé selon la revendication 1 **caractérisé en ce qu'**il est choisi parmi les composés suivants :
* Cis 2-[4-[4-(3-chloro-phényl)-pipérazin-1-yl]-cyclohexyl]-4-méthyl-2*H*-[1,2,4]triazi-ne-3,5-dione fumarate
* Trans 2-[4-[(2,3-dihydro-benzo[1,4]dioxin-2-ylméthyl)-amino]-cyclohexyl]-4-méthyl-2*H*-[1,2,4]triazine-3,5-dione hemifumarate
* Cis 4-méthyl-2-[4-(4-pyrimidin-2-yl-pipérazin-1-yl)-cyclohexyl]-2*H*-[1,2,4]triazine-3,5-dione
* Trans 4-méthyl-2-[4-(4-pyrimidin-2-yl-pipérazin-1-yl)-cyclohexyl]-2*H*-[1,2,4]triazi-ne-3,5-dione
* Trans 4-méthyl-2-[4-[4-(4,6diméthyl-pyrimidin-2-yl)-pipérazin-1-yl]-cyclohexyl]*-*2*H-*[1,2,4]triazine-3,5-dione
* Trans 4-méthyl-2-[4-[4-(4-méthyl-pyrimidin-2-yl)-pipérazin-1-yl] -cyclohexyl]-2*H*-[1,2,4]triazine-3,5-dione
* Trans 2-[4-[4-(4-chloro-pyrimidin-2-yl)-pipérazin-1-yl]-cyclohexyl]-4-méthyl-2*H*-[1,2,4]triazine-3,5-dione
* Trans 2-[4-[4-(5-fluoro-pyrimidin-2-yl)-pipérazin-1-yl]-cyclohexl]-4-méthyl-2*H*-[1,2,4]triazine-3,5-dione
* Trans 2-méthyl-5-[4-(4-pyrimidin-2-yl-pipérazin-1-yl)-cyclohexyloxy]-2*H*-[1,2,4]-triazin-3-one
* Trans 2-méthyl-5-[4-[4-(4-méthyl-pyrimidin-2-yl)-pipérazin-1-yl]-cyclohexyloxy]-2*H*-[1,2,4]triazin-3-one
* Trans 2-méthyl-5-[4-[4-(4,6-diméthyl-pyrimidin-2-yl)-pipérazin-1-yl]-cyclohexyl-oxy]-2*H*-[1,2,4]triazin-3-one
* Trans 2-méthyl-5-[4-[4-(4-chloro-pyrimidin-2-yl)-pipérazin-1-yl]-cyclohexyloxy]-2*H*-[1,2,4]triazin-3-one
* Trans 2-méthyl-5-[4-(4-(4-méthoxy-pyrimidin-2-yl)-pipérazin-1-yl]-cyclohexyloxy]-2*H*-[1,2,4]triazin-3-one
* Trans 2-méthyl-5-[4-[4-(4-trifluorométhyl-pyrimidin-2-yl)-pipérazin-1-yl]-cyclohexyl-oxy]-2*H*-[1,2,4]triazin-3-one
* Trans 2-méthyl-5-[4-[4-(5-fluoro-pyrimidin-2-yl)-pipérazin-1-yl]-cyclohexyloxy]-2*H*-[1,2,4]triazin-3-one
* Trans 5- [4-[(2,3-dihydro-benzo[1,4]dioxin-2-ylméthyl)-amino]-cyclohexyloxy]-2-mé-thyl-2*H*-[1,2,4]triazin-3-one
* Trans 6-({4-[4-(3-chloro-phényl)-pipérazin-1-yl]-cyclohexyl}-méthyl-amino)-2,4-diméthyl-2*H*-[1,2,4]triazine-3,5-dione
* Cis 6-({4-[4-(3-chloro-phényl)-pipérazin-1-yl]-cyclohexyl}-méthyl-amino)-2,4-dimé-thyl-2*H*-[1,2,4]triazine-3,5-dione

3. Procédé de préparation des composés chimiques selon les revendications 1 et 2 où B représente un groupement IIIa ou IIIb **caractérisé en ce que** l'on traite un dérivé de formule générale IV 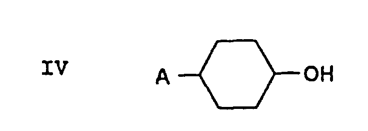 dans lequel A est tel que défini dans la revendication 1 avec un intermédiaire de type Va ou Vb 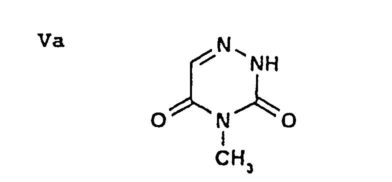 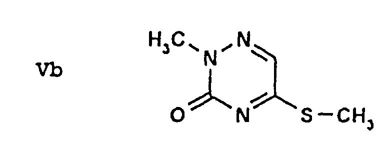 la condensation entre les dérivés IV et Va peut être effectuée selon les conditions de la réaction de Mitsunobu, et
la condensation entre les dérivés IV et Vb peut être effectuée en présence d'hydrure de sodium ou de tertiobutylate de potassium dans le dioxane ou le THF.

4. A titre de médicaments utilisables dans le traitement des maladies nécessitant des agonistes de récepteurs 5HT_{1A}, les composés définis selon l'une des revendications 1 et 2.

5. A titre de médicaments utiles, par exemple, pour le traitement de l'anxiété, la dépression, la douleur, la neurodégénérescence, la schizophrénie, la maladie d'Alzheimer, les troubles du sommeil, la régularisation de prise de nourriture, la régularisation de la sécrétion gastrique, le traitement des désordres vasculaires, cardiovasculaires et cérébrovasculaires, les composés définis selon l'une des revendications 1 et 2.

6. Composition pharmaceutique **caractérisée en ce qu'**elle contient à titre de principe actif un composé défini selon l'une des revendications 1 et 2.

7. Composition pharmaceutique selon la revendication 6, **caractérisée en ce qu'**elle contient un composé défini selon l'une des revendications 1 et 2 en association avec tout excipient approprié.

8. Composition pharmaceutique selon l'une des revendications 6 et 7, **caractérisée en ce qu'**elle contient un composé défini selon l'une des revendications 1 et 2 associé à un autre principe actif.

## Claims

1. Difunctionalized cyclohexane derivatives corresponding to the general formula I 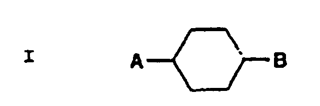 in which
- A represents a group of formula
• 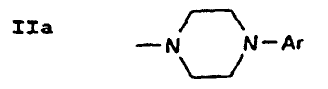 in which Ar itself represents a structure of aromatic type chosen from phenyl or pyrimidinyl, optionally substituted by one or more C₁₋₃ alkyl, C₁-C₃ alkoxy, trifluoromethyl or halogen groups,
• 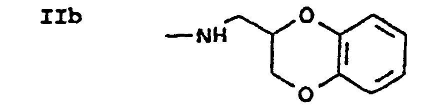
- B represents a heterocyclic group of formulae
. 3, 5-dioxo-(*2H*,*4H*)-1,2,4-triazine substituted at the 2 position, (IIIa) 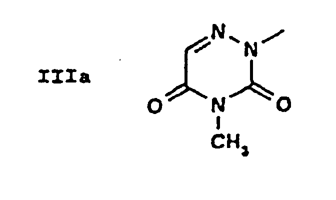
. 3-oxo-(*2H*)-1,2,4-triazine substituted at the 5 position, (IIIb) 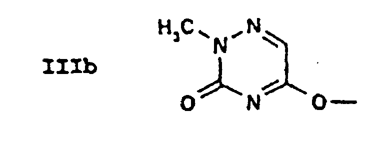 or:
. 3,5-dioxo-6-amino-(*2H*,*4H*)-1,2,4-triazine, (IIIc) 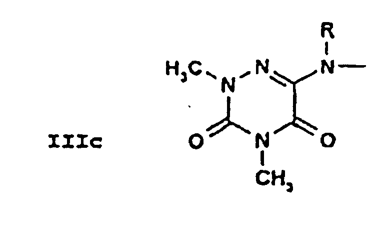
in which R represents a C₁-C₃ alkyl group,
and the salts of the compounds of general formula I with pharmaceutically acceptable acids,
it being possible for the compounds of formula I to be provided in the form of various "cis" and "trans" isomers and of various enantiomers of the compounds possessing asymmetric carbons.

2. Compound according to Claim 1, **characterized in that** it is chosen from the following compounds:
* cis-2-[4-[4-(3-Chlorophenyl)piperazin-1-yl]-cyclohexyl]-4-methyl-2*H*-[1,2,4]triazine-3,5-dione fumarate
* trans-2-[4-[(2,3-Dihydrobenzo[1,4]dioxin-2-ylmethyl)amino]cyclohexyl]-4-methyl-2*H*-[1,2,4]triazine-3,5-dione hemifumarate
* cis-4-Methyl-2-[4-(4-pyrimidin-2-yl-piperizin-1-yl)cyclohexyl]-2*H*-[1,2,4]triazine-3,5-dione
* trans-4-Methyl-2-[4-(4-pyrimidin-2-yl-piperizin-1-yl)cyclohexyl]-2*H*-[1,2,4]triazine-3,5-dione
* trans-4-Methyl-2-[4-[4-(4,6-dimethylpyrimidin-2-yl)piperizin-1-yl]cyclohexyl]-2*H*-[1,2,4]triazine-3,5-dione
* trans-4-Methyl-2-[4-[4-(4-methylpyrimidin-2-yl)piperazin-1-yl]cyclohexyl]-2*H*-[1,2,4]triazine-3,5-dione
* trans-2-[4-[4-(4-chloropyrimidin-2-yl)piperazin-1-yl]cyclohexyl]-4-methyl-2*H*-[1,2,4]triazine-3,5-dione
* trans-2-[4-[4-(5-Fluoropyrimidin-2-yl)piperizin-1-yl]cyclohexyl]-4-methyl-2*H*-[1,2,4]triazine-3,5-dione
* trans-2-Methyl-5-[4-(4-pyrimidin-2-yl-piperizin-1-yl)cyclohexyloxy]-2*H*-[1,2,4]triazin-3-one
* trans-2-Methyl-5-[4-[4-(4-methylpyrimidin-2-yl)piperazin-1-yl]cyclohexyloxy]-2*H*-[1,2,4]triazin-3-one
* trans-2-Methyl-5-[4-[4-(4,6-dimethylpyrimidin-2-yl)piperazin-1-yl]cyclohexyloxy]-2*H*-[1,2,4]triazin-3-one
* trans-2-Methyl-5-[4-[4-(4-chloropyrimidin-2-yl)piperazin-1-yl]cyclohexyloxy]-2*H*-[1,2,4]triazin-3-one
* trans-2-Methyl-5-[4-[4-(4-methoxypyrimidin-2-yl)piperazin-1-yl]cyclohexyloxy]-2*H*-[1,2,4]triazin-3-one
* trans-2-Methyl-5-[4-[4-(4-trifluoromethylpyrimidin-2-yl)piperazin-1-yl]cyclohexyloxy]-2*H*-[1,2,4]triazin-3-one
* trans-2-Methyl-5-[4-[4-(5-fluoropyrimidin-2-yl)piperazin-1-yl]cyclohexyloxy]-2*H*-[1,2,4]triazin-3-one
* trans-5-[4-[(2,3-dihydrobenzo[1,4]dioxin-2-ylmethyl)amino]cyclohexyloxy]-2-methyl-2*H*-[1,2,4]triazin-3-one
* trans-6-({4-[4-(3-Chlorophenyl)piperazin-1-yl]cyclohexyl}methylamino) -2,4-dimethyl-2*H*-[1,2,4]triazine-3,5-dione
* cis-6-({4-[4-(3-Chlorophenyl)piperazin-1-yl]cyclohexyl}methylamino)-2,4-dimethyl-2*H*-[1,2,4]triazine-3,5-dione.

3. Process for the preparation of the chemical compounds according to Claims 1 and 2 where B represents a group IIIa or IIIb, **characterized in that** a derivative of general formula IV 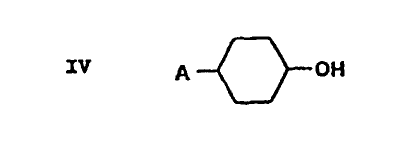 in which A is as defined in Claim 1, is treated with an intermediate of type Va or Vb 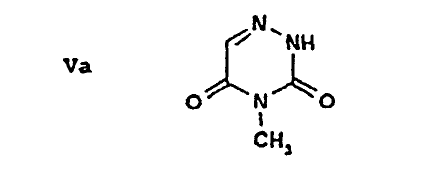 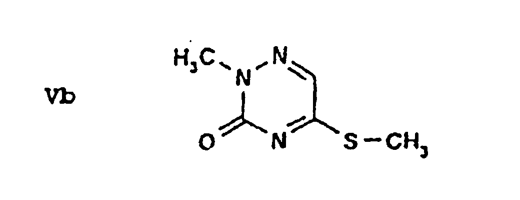 the condensation between the derivatives IV and Va can be carried out according to the conditions of the Mitsunobu reaction, and
the condensation between the derivatives IV and Vb can be carried out in the presence of sodium hydride or of potassium tert-butoxide in dioxane or THF.

4. As medicaments which can be used in the treatment of diseases requiring agonists of 5-HT_{1A} receptors, the compounds defined according to either of Claims 1 and 2.

5. As medicaments of use, for example, for the treatment of anxiety, depression, pain, neurodegeneration, schizophrenia, Alzheimer's disease and sleep disorders, the regulation of food intake, the regulation of gastric secretion and the treatment of vascular, cardiovascular and cerebrovascular disorders, the compounds defined according to either of Claims 1 and 2.

6. Pharmaceutical composition, **characterized in that** it comprises, as active principle, a compound defined according to either of Claims 1 and 2.

7. Pharmaceutical composition according to Claim 6, **characterized in that** it comprises a compound defined according to either of Claims 1 and 2 in combination with any appropriate excipient.

8. Pharmaceutical composition according to either of Claims 6 and 7, **characterized in that** it comprises a compound defined according to either of Claims 1 and 2 in combination with another active principle.

## Patentansprüche

1. Difunktionalisierte Cyclohexan-Derivate, die der allgemeinen Formel I 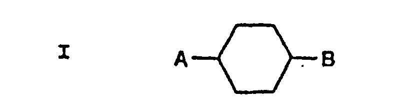 entsprechen, in der
· A eine Gruppe der Formel 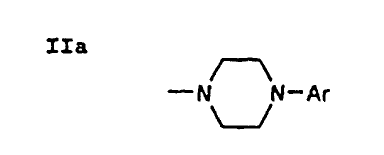 in der Ar selbst eine Struktur vom aromatischen Typ darstellt, welche ausgewählt ist aus Phenyl oder Pyrimidinyl, das gegebenenfalls mit einer oder mehreren (C₁-C₃)-Alkyl-, (C₁-C₃)-Alkoxy-, Trifluormethyl- oder Halogengruppen substituiert ist, 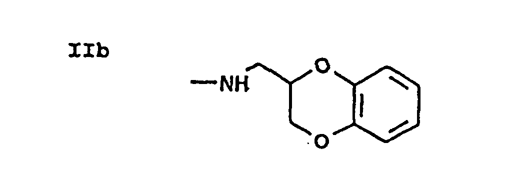 ist,
· B eine heterocyclische Gruppe der Formeln
. 3,5-Dioxo-(2H,4H)-1,2,4-triazin, das in Position 2 substituiert ist (IIIa) 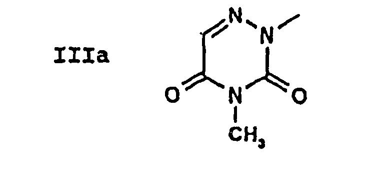
. 3-Oxo-(2H)-1,2,4-triazin, das in Position 5 substituiert ist (IIIb) 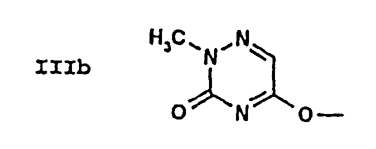 oder auch:
. 3,5-Dioxo-6-amino-(2H,4H)-1,2,4-triazin (IIIc) ist, 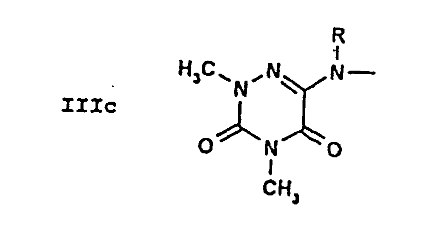 worin R für eine (C₁-C₃)-Alkylgruppe steht,
sowie die Salze der Verbindungen der allgemeinen Formel I mit pharmazeutisch annehmbaren Säuren,
wobei die Verbindungen der Formel I in der Form von verschiedenen "cis"und "trans"-Isomeren sowie von verschiedenen Enantiomeren der Verbindungen, die asymmetrische Kohlenstoffe besitzen, vorliegen können.

2. Verbindung nach Anspruch 1, **dadurch gekennzeichnet, dass** sie aus den folgenden Verbindungen ausgewählt ist:
· cis-2-[4-[4-(3-Chlorphenyl)piperazin-1-yl]cyclohexyl]-4-methyl-2H-[1,2,4]triazin-3,5-dionfumarat;
· trans-2-[4-[(2,3-Dihydrobenzo[1,4]dioxin-2-ylmethyl)amino]cyclohexyl]-4-methyl-2H-[1,2,4]triazin-3,5-dionhemifumarat;
· cis-4-Methyl-2-[4-(4-pyrimidin-2-ylpiperazin-1-yl)cyclohexyl]-2H-[1,2,4]triazin-3,5-dion;
· trans-4-Methyl-2-[4-(4-pyrimidin-2-ylpiperazin-1-yl)cyclohexyl]-2H-[1,2,4]triazin-3,5-dion;
· trans-4-Methyl-2-[4-[4-(4,6-dimethylpyrimidin-2-yl)piperazin-1-yl]cyclohexyl]-2H-[1,2,4]triazin-3,5-dion;
· trans-4-Methyl-2-[4-[4-(4-methylpyrimidin-2-yl)piperazin-1-yl]cyclohexyl]-2H-[1,2,4]triazin-3,5-dion;
· trans-2-[4-[4-(4-Chlorpyrimidin-2-yl)piperazin-1-yl]cyclohexyl]-4-methyl-2H-[1,2,4]triazin-3,5-dion;
· trans-2-[4-[4-(5-Fluorpyrimidin-2-yl)piperazin-1-yl]cyclohexyl]-4-methyl-2H-[1,2,4]triazin-3,5-dion;
· trans-2-Methyl-5-[4-(4-pyrimidin-2-ylpiperazin-1-yl)cyclohexyloxy]-2H-[1,2,4]triazin-3-on;
· trans-2-Methyl-5-[4-[4-(4-methylpyrimidin-2-yl)piperazin-1-yl]cyclohexyloxy]-2H-[1,2,4]triazin-3-on;
· trans-2-Methyl-5-[4-[4-(4,6-dimethylpyrimidin-2-yl)piperazin-1-yl]cyclohexyloxy]-2H-[1,2,4]triazin-3-on;
· trans-2-Methyl-5-[4-[4-(4-chlorpyrimidin-2-yl)piperazin-1-yl]cyclohexyloxy]-2H-[1,2,4]triazin-3-on;
· trans-2-Methyl-5-[4-[4-(4-methoxypyrimidin-2-yl)piperazin-1-yl]cyclohexyloxy]-2H-[1,2,4]triazin-3-on;
· trans-2-Methyl-5-[4-[4-(4-trifluormethylpyrimidin-2-yl)piperazin-1-yl]cyclohexyloxy]-2H-[1,2,4]triazin-3-on;
· trans-2-Methyl-5-[4-[4-(5-fluorpyrimidin-2-yl)piperazin-1-yl]cyclohexyloxy]-2H-[1,2,4]triazin-3-on;
· trans-5-[4-[(2,3-Dihydrobenzo[1,4]dioxin-2-ylmethyl)amino]cyclohexyloxyl-2-methyl-2H-[1,2,4]triazin-3-on;
· trans-6-({4-[4-(3-Chlorphenyl)piperazin-1-yl]cyclohexyl}methylamino)-2,4-dimethyl-2H-[1,2,4]triazin-3,5-dion;
· cis-6-({4-[4-(3-Chlorphenyl)piperazin-1-yl]cyclohexyl}methylamino)-2,4-dimethyl-2H-[1,2,4]triazin-3,5-dion.

3. Verfahren zur Herstellung von chemischen Verbindungen gemäß den Ansprüchen 1 und 2, worin B eine Gruppe IIIa oder IIIb darstellt, **dadurch gekennzeichnet, dass** man ein Derivat der allgemeinen Formel IV 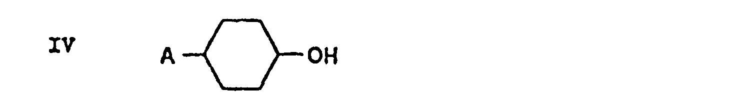 in der A wie in Anspruch 1 definiert ist, mit einem Zwischenprodukt des Typs Va oder Vb 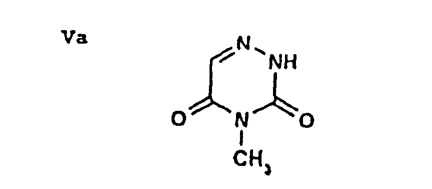 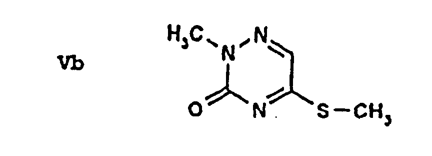 behandelt, wobei die Kondensation zwischen den Derivaten IV und Va gemäß den Bedingungen der Mitsunobu-Reaktion bewirkt werden kann und die Kondensation zwischen den Derivaten IV und Vb in Anwesenheit von Natriumhydrid oder Kaliumtertiärbutanolat in Dioxan oder THF bewirkt werden kann.

4. Verbindungen, definiert nach einem der Ansprüche 1 und 2, als Medikamente, die bei der Behandlung von Krankheiten verwendbar sind, welche Agonisten der 5HT_{1A}- Rezeptoren erfordern.

5. Verbindungen, definiert nach einem der Ansprüche 1 und 2, als Medikamente, die beispielsweise für die Behandlung von Angst, Depression, Schmerz, Neurodegeneration, Schizophrenie, Alzheimer-Krankheit, Schlafstörungen, die Regulierung der Nahrungsaufnahme, die Regulierung der Magensekretion, die Behandlung von vaskulären, kardiovaskulären und cerebrovaskulären Störungen nützlich sind.

6. Pharmazeutische Zusammensetzung, **dadurch gekennzeichnet, dass** sie als Wirkstoff eine Verbindung, definiert nach einem der Ansprüche 1 und 2, enthält.

7. Pharmazeutische Zusammensetzung nach Anspruch 6, **dadurch gekennzeichnet, dass** sie eine Verbindung, definiert nach einem der Ansprüche 1 und 2, in Verbindung mit irgendeinem geeigneten Träger enthält.

8. Pharmazeutische Zusammensetzung nach einem der Ansprüche 6 und 7, **dadurch gekennzeichnet, dass** sie eine Verbindung, definiert nach einem der Ansprüche 1 und 2, in Verbindung mit einem anderen Wirkstoff enthält.
